# EUROPEAN PATENT APPLICATION

(11) **EP 1 972 332 A1**
(43) Date of publication of application: **24.09.2008**
(21) Application number: 07006042.1
(22) Date of filing: 23.03.2007
(51) Int. Cl.: A61K 9/00, A61K 9/20, A61K 9/22, A61K 47/38, A61K 47/42

(54) **Mucosal bioadhesive slow release carrier for delivering active principles**

(71) Applicant: BioAlliance Pharma, 75015 Paris (FR)
(72) Inventor: Lemarchand, Caroline, 75014 Paris (FR)
(74) Representative: Vaillant, Jeanne

(57) **Abstract**

A mucosal bioadhesive slow release carrier comprising an active principle, which can release the active principal for a duration of longer than 20 hours. This bioadhesive carrier contains at least one bioadhesive natural protein of vegetal origin and at least one sustained release polymer, as well as a method for its preparation.

## Description

### 1. Field of the Invention

The present invention relates to a bioadhesive slow release carrier for the extended and controlled release of an active principle that can be used on mucosal surfaces. A process for manufacturing the bioadhesive system, a method for delivering an active ingredient to a mammal, as well as methods of treating, curing or preventing various medical conditions are also disclosed.

### 2. Background of the Invention and Related Prior Art

The natural protein of vegetable origin, like the pea protein, is well known for their nutritive and highly digestible qualities. However, pea protein has never been used in drugs.

Mucous membranes are linings of ectodermic origin, covered in epithelium, and are involved in absorption and secretion. They line various body cavities that are exposed to the external environment as well as internal organs, such as the nostrils, the lips, the ears, the genital area, the digestive tract and the anus. Parts of the body featuring mucous membranes include most of the respiratory tract and the entire gastrointestinal tract, as well as the vagina, cervix, the clitoris, the covering of the glans penis and the inside of the prepuce. Many of the afore mentioned mucous membranes secrete mucus, which is a viscous colloid containing antiseptic enzymes such as lysozymes and immunoglobulins and is made up of mucins and inorganic salts suspended in water.

One of the problems associated with a bioadhesive drug delivery system for mucous membranes is that the lubricious nature of the mucous membranes allows for the active substance to be washed away or diluted lowering the drugs bioavailability such that the administered drug does not effectively treat the medical condition at hand. Another problem is that in the oral cavity, eating drinking and speaking and the constant replacement of the saliva often effects the delivery of the active substance.

Oral mucosa bioadhesive delivery systems are also well known in the art and arc used to treat various medical conditions. These delivery systems are generally made of water soluble carbomers or insoluble polymers that can contain maize starch. Generally the delivery of the active principle is over a period of less than 11 hours. Thus, in these drug delivery systems the carrier has to be replaced at least twice a day.

For instance, U.S. Patent No. 5,643,603 describes a composition of a bloadhesive sustained delivery carrier for drug administration, which composition is made up of a mixture of pregalatinized starch and a synthetic polymer such as polyacrylic acid, hydroxyethyl cellulose, carboxymethyl cellulose, PVA, PVP and PEG (95%) and a drug (0.1 to 5%). This bioadhesive can be adhered to the mucosa or teeth and was deemed to deliver the drug over a period of seven hours.

Another form of a bioadhesive tablet is described in U.S. Patent No. 6,248,358 in which the active ingredient is protected from water and the surrounding environment. This tablet contains 5 to 50% by weight hydroxypropyl methyl cellulose, 0.5 to 25% by weight corn starch, 1 to 50% by weight lactose, 0.5 to 10% by weight water insoluble cross-linked polycarbophil and 1 to 75% by weight carbomer or carbomer 974P. This hydrated sustained release tablet can deliver the active ingredient to the bloodstream via the patient's vaginal or buccal cavity.

A bioadhesive solid dosage form is described in U.S. Patent No. 6,303,147, which has 0.001 to 10% of an active ingredient, 80 to 98% of pregelatinized starch, 1 to 10% of a hydrophilic matrix forming polymer such as polyacrylic acid, carbomer, hydroxyethyl cellulose, HPMC, carboxymethylcellulose, PVA or mixtures of these hydrophilic polymers, 0.2 to 5% of sodium stearyl fumarate and 0 to 1 % of a glidant. It can be used for buccal, nasal, rectal or vaginal administration. The adhesion time of these tablets is about nine hours.

U.S. Patent No. 6,916,485 describes a prolonged release bioadhesive therapeutic system containing 10 to 2,000 mg active ingredient, 50% by weight natural proteins, at least 20% by weight of milk protein concentrate, 10 to 20% by weight of a hydrophilic polymer, a compression excipient such as corn starch, lactose or polyol and 3.5 to 10% by weight alkali metal alkylsulfate. This system is a mucoadhesive tablet for delivery to the mucosa.

All of the above issued patents use carbopol with starch or natural milk proteins or bioadhesive agents, which play a role In the adhesion of the tablet.

However, in many of the above-mentioned slow release bioadhesive systems, the formulation of low aqueous soluble or insoluble active principles is difficult; in particular when these systems use the carbopol with starch as a bioadhesive agent since the carbopol is difficult to formulate. Various categories of medicinal agents such as antivirals, analgesics, anti-inflammatories, antibiotics and antiseptics have members, which are hard to formulate and administer due to their low aqueous solubility or insolubility. An example of an antiseptic with low aqueous solubility is iodine, which crystallizes when placed in water. Another example of an insoluble analgesic that is difficult to formulate is fentanyl base. Numerous antiviral and immunosuppressive agents such as acyclovir are also difficult to formulate, have poor percutaneous penetration and have complications arising due to intramuscular administration at a strongly alkaline pH of 10-11.

Morever, the use of an excipient of animal origin can lead to a viral security issue.

In addition, the use of milk derivatives is excluded for people allergic to milk or milk derivatives.

For instance, the oral absorption of acyclovir is highly variable with a bioavailability ranging from 15% and 30%. In patients the systemic treatment regimen is 200 mg tablets, five times a day. Moreover, after systemic administration of acyclovir orally, peak acyclovir concentrations are found in saliva at the lower end of the 50% inhibitory dose of herpes simplex-1 virus. Local treatment of acyclovir as a cream is also poor due to poor percutaneous absorption. This cream must be applied at least 5 times a day over a period of 5 days.

Patients being treated for malignant diseases or HIV have further oral complications associated with their particular disease, due to immunosuppression. For instance, some of the most common oral manifestations in people who are infected with AIDS include bacterial infections such as gingivo-periodontal disease fungal infections such as Candidiasis, viral infections such as Epstein-Barr virus, oral hairy leukoplakia, herpes simplex-1 virus, variacella-Zoster virus, human papilloma virus, cytomegalovirus, neoplasms such as Kaposi's sarcoma and lymphoma and other oral lesions including oral ulcers and salivary gland enlargement. Oral pain may be associated with each one of these complications.

Likewise, people undergoing chemotherapy and head/neck radiation also have oral complications including mucositis, infection, salivary gland dysfunction, taste dysfunction, viral, fungal and bacterial infections, xerostomia and gastrointestinal mucositis caused by the secondary modifications in the oral cavity. In about 40% of patients undergoing chemotherapy ulcerative oral mucositis occurs. In patients undergoing neck irradiation ulcerative oral mucositis occurs in almost every case.

Therefore, there is a need to accommodate multiple drugs treating different medical complications in a single drug delivery system to avoid multiple administration of different medicaments.

Thus, there are problems associated in the prior art for mucosal delivery that can deliver the active principle to treat various medical complications over a long period of time. Furthermore there are problems also associated with the prior art with respect to the delivery of active principles, which have low aqueous solubility or are insoluble. Moreover, there is a need in the art to diminish taking multiple drugs to treat different medical conditions.

Therefore it is an object of the present invention to overcome the deficiencies in the prior art bioadhesive delivery systems.

It is an object of the present invention to provide a bioadhesive slow release carrier for mucosal delivery of an active ingredient over a long period of time.

It is another object of the present invention to provide a bioadhesive slow release carrier for mucosal delivery of a soluble or insoluble active principle.

It is yet another object of the present invention to provide a mucosal delivery bioadhesive slow release carrier In which the active principle can be administered once a day.

Another object if the present invention is to provide a mucosal bioadhesive slow release carrier in which the active principle can be liberated immediately and then liberated over a prolonged period of time.

It is yet another object of the present invention to provide a mucosal bioadhesive slow release carrier that can contain at least two different active principles.

It is yet another object of the present invention to provide a mucosal bioadhesive slow release carrier that can contain a bioadhesive agent of vegetal origin.

It is another object of the present invention to provide a process for manufacturing a mucosal bioadhesive slow release carrier that can deliver a soluble or insoluble active principle.

Yet another object of the present invention is a method for delivering an active principle to a mammal.

Methods of treating, curing or preventing various medical conditions and diseases are also objects of the present invention.

Methods of treating, curing or preventing mucosal diseases such as buccal and genital diseases are also objects of the present invention.

Use of the mucosal bioadhesive slow release carrier for the manufacture of medicaments to treat, cure or prevent certain diseases is also an object of the present invention.

These and other objects are achieved by the present invention as evidenced by the summary of the invention, description of the preferred embodiments and the claims.

### SUMMARY OF THE INVENTION

The present invention relates to a mucosal bioadhesive slow release carrier comprising at least one active principle, at least one bioadhesive natural protein of vegetal origin and at least one polymer that provides sustained release of the active principle.

In another aspect, the present invention relates to a mucosal bioadhesive slow release carrier comprising at least one active principle, a diluent, an alkali metal alkylsulfate, a binding agent, at least one bioadhesive natural protein of vegetal origin and at least one polymer that provides sustained release of the active principle.

In another aspect the present invention provides a mucosal bioadhesive slow release carrier comprising a wetting agent comprising at least one active principle comprising 5 to 80% by weight of at least one bioadhesive natural protein of vegetal origin and mixtures thereof and 5% to 80% by weight of at least one polymer that provides a sustained release of the active principle.

In another aspect the present invention provides a mucosal bioadhesive slow release carrier comprising a wetting agent comprising at least one active principle comprising 1 to 75 % by weight of a diluent, and 1 to 10 % by weight of an alkali metal alkylsulfate, and further comprising 0.5 to 5 % by weight of a binding agent and 5 to 80 % by weight of at least one bioadhesive natural protein of vegetable origin and mixtures thereof and 5% to 80% by weight of at least one polymer that provides a sustained release of the active principle.

The at least one natural protein from vegetable origin that can be used in the present invention include natural pea proteins, natural soy proteins, natural potato proteins, natural sweet potato proteins, natural wheat proteins, gliadin proteins and mixtures thereof.

The at least one active principle that can be used in the present invention include analgesics, anaesthetics such as lidocain, antalgics, antiviral agents, anti-inflammatory agents, antiemetic agents, antibiotics and antiseptics. Two or more different active principles besides the ones mentioned above including antiviral agents with different spectrum than those described above, an antifungal agent and a salivary agent can also be formulated in the bioadhesive carrier of the present invention.

In yet another aspect the present invention provides a method for delivering an active principle to a mammal, said method comprising mucosally administering to a mammal in need of said active principle, a bioadhesive slow release carrier comprising al least one active principle, at least one bioadhesive natural protein of vegetal origin and at least one polymer that provides sustained release of the active principle. This bioadhesive slow release carrier can also comprise a diluent, an alkali metal alkylsulfate and a binding agent.

In yet another aspect the present invention provides a method for delivering an active principle to a mammal, said method comprising mucosally administering to a mammal in need of said active principle, a bioadhesive slow release carrier comprising a wetting agent comprising at least one active principle, at least one bioadhesive natural protein of vegetal origin and mixtures thereof and 5 to 80% by weight of at least one polymer that provides a sustained release of the active principle. This bioadhesive slow release carrier can also comprise 1 to 75 % by weight of a diluent and 1 to 10 % by weight of an alkali metal alkylsulfate and further comprises 0.5 to 5 % by weight of a binding agent.

In another aspect the invention provides the use of natural protein of vegetal origin for the manufacture of a mucosal bioadhesive slow release carrier.

In another aspect the invention provides the use of natural pea protein for the manufacture of a mucosal bioadhesive slow release carrier.

Methods of alleviating, treating, preventing or curing various medical conditions also are a part of the present invention. Thus, medical conditions or diseases such as orofacial herpes-herpes simplex virus 1 (HSV-1), genital herpes-herpes simplex virus 2 (HSV-2), oral mucositis, fungal infections such as Candidiasis, viral infections such as Epstein-Barr virus, oral hairy leukoplakia, variacella-Zoster virus, human papilloma virus, cytomegalovirus, Kaposi's sarcoma due to human herpes V8 and genital warts due to human papilloma virus and other oral lesions including oral ulcers and salivary gland disturbance, altered oral flora (decreased bacterial flora), taste dysfunction, periodontitis, xerostomia (salivary gland dysfunction), gastrointestinal mucositis causing secondary changes in oral status including inflammation, hygiene and dietary intact and oral pain can be treated using the mucosal bioadhesive slow release carrier of the present invention.

The methods of alleviating, treating, preventing or curing the above medical conditions using the mucosal bioadhesive slow release carrier of the present invention can be used to treat immunocompromised mammals.

Use of the mucosal bioadhesive slow release carrier of the present invention for the manufacture of a medicament to alleviate, treat, prevent or cure mucosal diseases or buccal diseases or genital diseases is also provided in the present invention.

### BRIEF DESCRIPTION OF THE DRAWINGS

Fig. 1 is a schematic representation of one of the processes of the present invention.

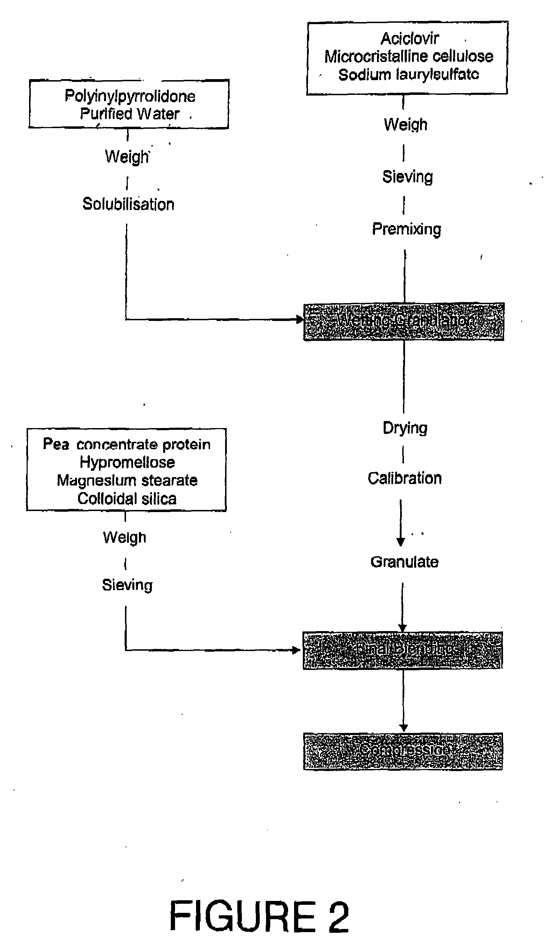

Fig. 2 is a schematic representation of another one of the processes of the present invention.
Fig. 3 is a graph showing the adhesion force of the bioadhesive slow release carrier of the present invention using different bioadhesive agents.

### DESCRIPTION OF THE PREFERRED EMBODIMENTS OF THE INVENTION

As used herein the term "mucosal" encompasses oral/ buccal, vaginal, esophageal, nasal and rectal delivery.

The term "bioadhesive" refers to any adhesive that interfaces with living tissue and/or biological fluid.

By the term "carrier" is meant any object that can transport at least one active principle.

As used herein the term "active principle", "drug" and "active ingredient" are used interchangeably. The active principle is used to alleviate, treat or prevent a medical condition or disease. In some instances the active principle can be used to cure a medical condition or disease.

"Medical condition" and "disease" are also used interchangeably herein and refer to any condition in a mammal or one of its parts that impairs normal functioning in the mammal. This impairment may lead to illness or sickness characterized by certain symptoms and physical findings suffered by a mammal.

The term "mammal" encompasses any of various warm-blooded vertebrate animals of the class Mammalia, including humans, characterized by a covering of hair on the skin and, in the female, milk-producing mammary glands for nourishing the young.

As used herein the term "buccal" means of, relating to, involving or lying in the mouth.

As used herein the term "diluent" means a diluting agent and encompasses such agents that are insoluble diluents and soluble diluents.

The term "binder" when used herein relates to any pharmaceutically acceptable film which can be used to bind together the active and inert components of the carrier together to maintain cohesive and discrete portions. Binders provide the matrix from which the active principle is gradually secreted.

As used herein, the expression "residence time" will refer to the time for which the carrier placed on the target mucosal surface will remain substantially intact.

Similarly, throughout the text, the expression "slow release" or "sustained release" are used interchangeably and mean that the active principle is released immediately after 30 minutes and then over a prolonged period of time of at least 15 hours or at least 18 hours or at least 20 hours or at least 24 hours and up to 36 hours.

By the term "insoluble" when referring to the active principle means that the drug can be totally insoluble in an aqueous medium or has limited solubility in an aqueous medium.

By "limited solubility" is meant that the active principle has a solubility below 10 mg/ml in 250 ml of water over a pH range from 1.0 to 7.5. Classes of drugs that can have "limited solubility' are those drugs that are hydrophobic or those which are classified in the Biopharmaceutical Classication System (BCS) as being type III or type IV drugs.

As used herein "of vegetal origin" means any protein that can be found in plants that acts as a bioadhesive and thus interfaces with living tissue and/or biological fluid.

More specifically, the present invention relates to a mucosal bioadhesive slow release carrier that can deliver an active principle over a long period of time. For example, the active principle can be delivered from 30 minutes to 15 hours, 30 minutes to 18 hours, 30 minutes to 20 hours or 30 minutes to 24 hours or 30 minutes to 36 hours. Thus, this carrier has the advantage that it provides an effective residence time for the active principle such that only a single daily dosage is necessary.

More specifically, the bioadhesive slow release carrier of the invention can be used for preventing, alleviating, curing or treating general diseases or mucosal diseases, buccal, oesophageal or vaginal infections in mammals.

Still more specifically, the bioadhesive slow release carrier of the invention can be used for preventing, alleviating, curing or treating buccal herpes simplex 1 (HSV-1) infections, especially in immunodepressed mammals such as those mammals that have AIDS or SIV or have been subjected to chemotherapy or radiation therapy.

The present invention is not limited to treating only humans, but also encompasses veterinary applications, especially since it is well known that animals also can have oral medical complications.

The process to formulate the bioadhesive slow release carrier of the present invention permits the use of an active principle, which can be soluble, insoluble or having limited solubility in an aqueous solution such as water. It is known in the art that insoluble nr limited solubility drugs pose problems in their formulation and ' there is limited choice for a delivery system. In many delivery systems there is decreased bioavailability of the active principle, incomplete release from the dosage form and higher interpatient variation. Thus, in many instances the active principle must administered more frequently and monitored more closely. The present invention overcomes these difficulties.

The carrier of the present invention can be in the form of a tablet, microspheres and the like. They can be formulated in any shape such as rectangular, circular, square, oval and the like. It should be appreciated that the dimensions of the carrier depend on the delivery mode that is used. For example, for gingival delivery, the carrier has a flat surface and a curved surface. The carrier can also be coated with an active principle described hereafter.

The mucosal bioadhesive slow release carrier of the present Invention comprises at least one active principle, at least one bloadheslve natural protein of vegetal origin and at least one polymer that provides sustained release of the active principle. This mucosal bioadhesive slow release carrier can also comprise a diluent, an alkali metal alkylsulfate and a binding agent.

More specifically, mucosal bioadhesive slow release carrier comprises a wetting agent comprising at least one active principle, at 5 to 80% by weight of at least one bioadhesive natural protein of vegetal origin and mixtures thereof and 5% to 80% by weight of at least one polymer that provides a sustained release of the active principle. This mucosal bioadhesive slow release carrier can also comprise 1 to 75 % by weight of a diluent and 1 to 10 % by weight of an alkali metal alkylsulfate and further comprises 0.5 to 5 % by weight of a binding agent.

In another embodiment a mucosal bioadhesive slow release carrier comprising a wetting agent comprising at least one active principle comprising 1 to 75 % by weight of a diluent, and 1 to 10 % by weight of an alkali metal alkylsulfate, and further comprising 0.5 to 5 % by weight of a binding agent and 5 to 80 % by weight of at least one bioadhesive natural protein of vegetable origin and mixtures thereof and 5% to 80% by weight of at least one polymer that provides a sustained release of the active principle is provided.

The at least one natural protein of vegetal origin that can be used in the present invention include natural pea proteins, natural soy proteins, natural potato proteins, natural wheat proteins and gliadin proteins.

More specifically, the active principle which is incorporated into the bioadhesive slow release carrier includes antiviral agents, antifungals, analgesics, anaesthetics, antalgics, anti-inflammatory agents, antiemetics, antibiotics and antiseptics. More than one active principle can be used depending on the needed application. For example, one can envision treating herpes simplex 1 using an antiviral such as, acyclovir, as well as an anti-inflammatory for pain in the same bioadhesive carrier.

Examples of antiviral agents that can be used in the bioadhesive carrier include, for example, vidarbine, acyclovir, ganciclovir, cidovir, valganciclovir, nucleoside analog reverse transcriptase inhibitors such as zidovudine, didanosine, zalcitabine, stavudine, lamivudine, non-nucleoside reverse transcriptase inhibitors such as nevirapine and delavridine, protease inhibitors such as saquinavir, ritonavir, indinavir, netfinavir, ribavirin, amantadine, rimantadine, releenza, tamiflu, pleconatil, penciclovir, famciclovir, foscarnet, interferons such as IFN-α and the like.

The antiviral agent is present in the bioadhesive carrier in a concentration between 10 to 200 mg. It can also be present in a concentration between 50 to 100 mg. It will be appreciated that the amount of the antiviral can vary depending on the mammal being treated as well as the medical condition of the mammal.

Examples of antifungal agents that can be used in the bioadhesive carrier include, for example, polyene antimycotic and imidazole and triazole such as clotrimazole.

The dosage of antifungal varies in the bioadhesive carrier with the antifungal utilized. Generally the does is between 10 mg to 150 mg.

Sulfa drugs and folic acid analogs, the beta-lactams including penicillins, vancomycin, ampicillin and amoxicillins and cephalosporins, aminoglycosides such as streptomycin, kanamycin, neomycin and gentamycin, tetracyclines such as doxycycline, macrolides, licosamides, streptogramins, fluoroquinolines such as ciprofloxacin, levofloxacin and moxifloxacin, polymixins, rifampin, mupirocin, cycloserine, aminocyclitols, glycopeptides and oxazolidinones are examples of antibiotics that can be used in the bioadhesive slow release carrier.

The dosage of antibiotic varies in the bioadhesive carrier with the antibiotic utilized. Generally the dose is between 10 mg to 150 mg.

Examples of anti-inflammatory drugs that can be used in the bioadhesive carrier include aspirin, salsalate, diflunisal, ibuprofen, ketoprofen, nabumetone, piroxicam, naproxen, diclotenac, indomethacin, sulindac, tolmetin, etodolac, ketorolac, oxaprozin, trisalicylate, acetaminophen, suprofen, corticoids, celecoxib and thaildomide.

The dose of the anti-inflammatory present in the bioadhesive slow release carrier is between 25 and 1,500 mg or between 50 and 500 mg depending on the anti-inflammatory utilized.

Sodium laurylsulfate, iodophore, iodine, chlorhexidine gluconate, quaternary ammonium compounds such as cetylpyridinium chloride, phenolic antiseptics such as Listerine®, povidone-iodine, hexetidine, triclosan, delmopinol, salifluor, sanguinarine, alkali metal akylsulfates and propolis are antiseptics that can be used in the present invention.

Between 0 to 5% of the antiseptic is used in the bioadhesive carrier of the invention.

Antiemetic drugs that can be used to treat nausea and vomiting, especially after chemotherapy include granisetron, ondansetron, dexamethasone and metoclopramide, 5-hydroxytryptanine (serotonin) inhibitors, dronabinol and prochloperazine and tropisetron. These drugs and combinations thereof can be used in the bioadhesive carrier.

The antiemetic is generally present in a dose between 1 to 100 mg in the carrier.

Besides the active principle, the bioadhesive slow release carrier has an adhesive system, which allows the carrier to adhere to mucosal surfaces over a prolonged period of time. The adhesive system comprises at least one bioadhesive natural protein of vegetal origin and at least one sustained release polymer. The adhesive system can also comprise a diluent, an alkali metal alkysulfate and a binding agent.

The diluent used in the present invention can be insoluble or soluble. The diluent used is insoluble when the active principle is soluble and the diluent is soluble when the active principle is insoluble.

Examples of insoluble diluents include microcrystalline cellulose, silicified microcrystalline cellulose, hydroxymethylcellulose, dicalcium phosphate, calcium carbonate, calcium sulfate, magnesium carbonate, tricalcium phosphate and the like.

Examples of soluble diluents include mannitol, glucose, sorbitol, maltose dextrates, dextrins, dextrose, lactose, starch and the like.

The diluent is present in an amount between 1 and 75 % by weight in the bioadhesive slow release carrier.

An alkali metal alkylsulfate is also a component of the bioadhesive carrier of the present invention. This alkali metal sulfate increases the granulation of the active principle acting as a solubilization agent. The alkali metal alkylsulfate that can be used in the formulation includes magnesium lauryl sulfate, potassium lauryl sulfate, sodium laurylsulfate and diethylsulphosuccinate. Generally it is present in the bioadhesive carrier at a concentration of between 1 to 10% by weight or 2 to 10% by weight.

The mucosal bioadhesive slow release carrier used in the present invention permits liberation of the active principle, preferably a prolonged and/or immediate and/or local liberation of the active principle.

The mucosal bioadhesive slow release carrier used in the present invention permits absorption of the active principle, preferably over a prolonged period.

The binders used In the present invention can be selected from carboxy vinyl polymer, methycellulose, hydroxyethylcellulose, hydroxypropyl cellulose, polyvinylpyrrolidone, polyethylene glycol and the like. The binders are present in the amount of 0.5 to 5% by weight in the bioadhesive slow release carrier.

The bioadhesive natural proteins of vegetal origin are those vegetal proteins that have bioadhesive properties as defined herein. Examples of these proteins include, but are not limited to, natural pea proteins, natural soy proteins, natural potato proteins, natural wheat proteins and gliadin proteins .and mixtures thereof.

They are present in the bioadhesive carrier at a concentration of 5 to 80% by weight. The can also be present in an amount of 10% to 40% by weight.

Regarding the vegetable proteins, they can be obtained from pea, soy, potato, wheat, gliadin and/or mixtures of these proteins. The method for producing pea protein is described in WO 2007/017571.

Example of pea proteins are those titrating a minimum of 85% proteins such as Nutralys® sold by Roquette (France). Pea protein is particularly rich in lysine and has high levels of branched amino acids, glutamic acid and arginine.

The sustained release polymers that can be used in the bioadhesive carrier include hydrophilic polymers including polysaccharides such as cellulose ethers, xanthum gum, scleroglucan, locust bean gum, gum Arabic, gum tragacanth, carob, alginic acid, alginates, carrageenates, agar-agar and guar gum either alone or in mixtures thereof. Other polymers that can be used in the present invention include cellulose based polymers such as hydromellose, cellulose acetate, cellulose esters, cellobiose, cellulose resins alone or in mixtures thereof.

The sustained release polymers are present in a concentration of 5% to 80% by weight. They can also be present in an amount of 10% to 40% by weight.

The alkali metal alkylsulfale are present in a concentration of 1 to 10 % by weight . They can also be present in an amount of 2% to 6% by weight.

Salivation agents such as pilocarpin and bethanechol and flavorings agents can also be added. Flavoring agents include calcium citrate, Safrole, and sweetening agents such as aspartame, cyclamates, saccharin and xylitol. Additionally compression excipients such as flow aids including talc, colloidal silicone dioxide, colloidal silica and lubricants including magnesium stearate, stearic acid, polyethylene glycol can also be added to the bioadhesive slow release carrier at the stage of blending.

These additional agents can be added to the carrier in the concentration range of 0.1 to 10% by weight of the total weight of the components in the carrier.

In one embodiment the bioadhesive slow release carriers are intended to prevent and treat HSV-1, HSV-2, varicella zoster virus (VZV), Epstein-Barr virus, human herpes virus 8, avian influenza, mumps, HIV, respiratory syncitial virus, influenza, parainfluenza and cytomegalovirus infections. A preferred active principle is a compound from the spectrum of nucleosides antivirals preferably selected from acyclovir, valacyclovir, ganciclovir or zidovudine. Thus in one aspect the nucleoside antiviral compound used in the bioadhesive is acyclovir, present in a dose of 10 to 200 mg per carrier.

In yet another embodiment the present invention relates to a mucosal bioadhesive slow release carrier comprising a wetting agent comprising 10 to 200 mg of an antiviral agent selected from the group of acyclovir, valacyclovir, gancyclovir and zidovudine, 1 to 75 % by weight of a diluent of microcrystalline cellulose and 2 to 10 % by weight of sodium lauryl sulphate and further comprising 0.5 to 5 % by weight of a polyvinylpyrrolidine and 10 to 40 % by weight of at least one bioadhesive natural protein of vegetal origin and mixtures thereof and 10% to 40% by weight of hypromellose.

In addition, and especially in the case of treating two different medical conditions the active principle described above can be combined with an antiviral, an antifungal, an analgesic, an anaesthetic, an antalgic, an antiemetic, a salivation agent, an antiseptic, an anti-inflammatory, an antibiotic and mixtures thereof.

For example, if a patient has herpes simplex virus 1 and pain the combined use of acyclovir with an analgesic such as ibuprofen is particularly advantageous since HSV-1 infection is sometimes accompanied by labial or buccal pain.

Thus, the present invention provides a mucosal bioadhesive slow release carrier comprising a wetting agent comprising at least two active principles selected from the group of antiviral agents, analgesics, anaesthetics, antalgics, anti-inflammatory agents, antiemetics, antibiotics, antiseptics, an antiviral, an antifungal, a salivation agent,at least one bioadhesive natural protein of vegetal origin and mixtures thereof and at least one polymer that provides a sustained release of the active principle.

In another embodiment, the present invention provides a mucosal bioadhesive slow release carrier comprising a wetting agent comprising at least two active principles selected from the group of antiviral agents, analgesics, anaesthetics, antalgics, anti-inflammatory agents, antiemetics, antibiotics, antiseptics, an antiviral, an antifungal, a salivation agent,1 to 75 % by weight of a diluent and 1 to 10 % by weight of an alkali metal alkylsulfate and further comprising 0.5 to 5 % by weight of a binding agent and 5 to 80 % by weight of at least one bioadhesive polymer selected from the group of natural polymers wherein said natural polymers are natural proteins of vegetal origin, and mixtures thereof and 5% to 80% by weight of at least one polymer that provides a sustained release of the active principle.

The combination of at least two active principles is also important in treating oral complications that arise from chemotherapy and head and neck radiation due to direct lethal and sublethal damage to oral tissues. Moreover, many patients have decreased immune systems, which lead to problems in the healing of oral tissues. Thus complications arising with patients undergoing chemotherapy and radiation treatment include oral mucositis, oral viral, bacterial and fungal infections, salivary gland dysfunctions altered oral flora, taste dysfunctions, xerostomia and gastrointestinal mucositis, which causes secondary changes in oral status including laste, hygiene and dietary intact. Gastrointestinal mucositis causes nausea and vomiting. These complications require treatment with more than one drug. For example, one can use the analgesic fentanyl base, fentanyl citrate or sulfentanyl which are important in treating severe resistant pain in particular associated with cancer, and an antiemetic to treat nausea and vomiting. It will be appreciated that the combinations of the different active principles utilized are based on the medical conditions that the patient/mammal has and the treatment that is necessary.

Hence the present invention provides a bioadhesive slow release carrier that can be used in the treatment of oral complications due to chemotherapy or radiation treatment comprising a wetting agent containing at least two active ingredients selected from the group of antiviral agents, analgesics, anaesthetics, antalgics, anti-inflammatory agents, antiemetics, antibiotics, antiseptics, an antiviral, an antifungal, a salivation agent, at least one bioadhesive of natural origin and mixtures thereof and at least one polymer that provides a sustained release of the active principle.

The present invention provides a bioadhesive slow release carrier that can be used in the treatment of oral complications due to chemotherapy or radiation treatment comprising a wetting agent containing at least two active ingredients selected from the group of antiviral agents, analgesics, anaesthetics, antalgics, anti-inflammatory agents, antiemetics, antibiotics, antiseptics, an antiviral, an antifungal, a salivation agent, 1 to 75 % by weight of a diluent and 1 to 10 % by weight of an alkali metal alkylsulfate and further comprising 0.5 to 5 % by weight of a binding agent and 5 to 80 % by weight of at least one bioadhesive natural protein of vegetal origin and mixtures thereof and 5% to 80% by weight of at least one polymer that provides a sustained release of the active principle.

In one aspect the unique properties of the mucosal bioadhesive slow release carrier is due to its formulation. The processes of the present invention concerns using a wet granulation or dry granulation or direct compression technique in its formulation.

In another aspect the present invention provides a process comprising:
(a) mixing an active principle with natural proteins of vegetal origin representing between 50 and 100% by weight of the active principle with excipients and fillers comprising at least one hydrophilic polymer and
(b) mixing the mixture in (a) with an alkali metal alkylsulfate.

The concentration of the alkyl metal sulfate in the procedure set forth above is between 3.5 and 10% by weight of the prolonged release mucosal bioadhesive or it can be between 4 and 6% by weight.

In another aspect the present invention relates to a process for producing a mucosal bioadhesive slow release carrier comprising the steps of:
(a)mixing an active principle with natural proteins being between 50 to 100% by weight of the active principle and fillers comprising at least a hydrophilic polymer;
(b)wetting the mixture in (a) with water;
(c)drying the wetted mixture and calibrating the grains obtained after drying and;
(d) mixing the grains with at least one compression agent.

In another aspect the present invention relates to a process for producing a mucosal bioadhesive slow release carrier comprising the steps of:
(a)mixing an active principle with natural proteins being between 50 to 100% by weight of the active principle and fillers comprising at least a hydrophilic polymer;
(b)wetting the mixture in (a) with a monohydrate sugar or polyol ;
(c)drying the wetted mixture and calibrating the grains obtained after drying and;
(d) mixing the grains with a alkylsulfate of an alkaline metal.

The concentration of the alkyl metal sulfate in the procedure set forth above is between 3.5.and 10% by weight of the prolonged release mucosal bioadhesive or it can be between 4 and 6% by weight. The sucrose monohydrate can be lactose monohydrate.

In another aspect the present invention also concerns a process for preparing a mucosal controlled release bioadhesive therapeutic system containing at least one active principle dissolution percentage of more than 70% over 8 hours, comprising at least:
- mixing the active principle with natural proteins, these latter representing at least 50% by weight of active principle, and with excipients and fillers comprising at least one hydrophilic polymer; and
- mixing with an alkali metal alkylsulfate in a concentration in the range 3.5% to 10% by weight of the bioadhesive therapeutic system, preferably 4% to 6%.

In one implementation, the bioadhesive therapeutic system is a prolonged release mucosal bioadhesive tablet and the preparation of which comprises the following steps:
a) mixing the active principle with natural proteins, said proteins representing at least 50% by weight of the active principle, and with excipients and fillers comprising at least one hydrophilic polymer;
b) wetting the mixture obtained at a) with a monohydrate sugar or polyol type binder;
c) drying the mixture and sizing the grains obtained; and
d) mixing the grains obtained with an alkali metal alkylsulphate in a concentration in the range 3.5% to 10% by weight of tablet, preferably 4% to 6%.

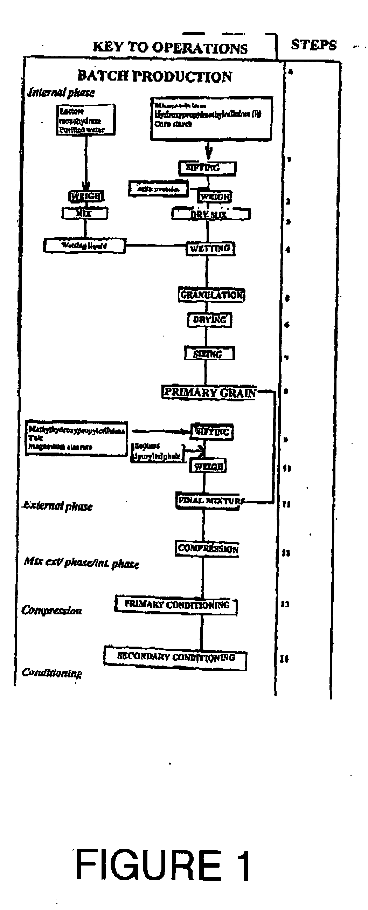

In the method of the invention, the active principle or active principles, if necessary mixed with hydroxypropylmethylcellulose or metolose (HPMC) is sieved with an open space of between 0.4 and 1 mm, then mixed with at least 50% by weight of natural proteins, such as pea proteins. The powder obtained is homogenized. It then undergoes wetting step in a granulation step in which the powder is mixed with a lactose or saccharose solution in distilled water. It is then followed by drying to produce a residual moisture content of about 3%. It is then followed by a calibration step, mixing with the remaining excipients and by a compression step. A particular feature of the wetting liquid, lactose in water, is that the quantity introduced during batch production is not fixed but should be determined as a function of the appearance of the grain. There are three possibilities:
- either all the wetting liquid is introduced and the subsequent production phases of the tablet are carried out conventionally by adding excipients, as indicated in the examples below;
- or not all of the wetting liquid is used and metolose is added with the formulation excipients to arrive at a final concentration (between 0.1% and 1%, preferably between 2% and 4% of the final tablet). A typical production diagram is shown in FIG. 1.
- or all of the lactose or saccharose is dissolved in half of the theoretical quantity of purified water. All of this wetting liquid is introduced into the mixture. The remaining half of the purified water is used as a reserve when optimising wetting and can be adjusted as a function of batch size.

A further characteristic of the method for preparing tablets of the invention is adding an alkali metal alkylsulfate in step d) above in a concentration in a range of 4% to 6%. It is actually added at the same time as the formulation excipients such as talc and magnesium stearate, to endow the tablets with the functions described above, in particular as regards rate of dissolution.

The invention also concerns a method for preparing a bioadhesive therapeutic system other than tablets, such as microspheres, in which a step for adding an alkali metal alkylsulfate in the same range of concentration as described above is added to the preparation.

The alkali metal alkylsulfate is preferably sodium laurylsulfate or sodium diethylsulphosuccinate. One of the production of pilot batches described below used sodium laurylsulfate in a concentration of 4.5%.

In an aspect the present invention also provides a method for preparing a mucosal bioadhesive slow release carrier comprising:
a) granulating a mixture of at least one active principle, a diluent and a binding agent;
b) blending said granulated mixture with at least one bioadhesive polymer, at least one sustained release polymer and at least one compression agent; and
c) compressing the blended mixture obtained in b).

In another aspect, the present invention also relates to a method of preparing a bioadhesive carrier, the method comprising:
a) mixing at least one active principle with an alkali metal alkylsulfate and a diluent to form a mixture;
b) wetting said mixture with a binding agent;
c) drying and calibrating said mixture;
d) granulating said mixture to form primary granules;
e) blending said primary granules with at least one bloadhesive polymer and at least one sustained release polymer and at least one compression agent; and
f) compressing the blended mixture obtained in e).

More specifically, the active principle, the diluent and the alkali metal alkylsulfate are first individually weighed, sieved and promixed in a blender, The binding agent is weighed and solubilized using purified water. The solubilized binding agent is then added to the mixture containing the active ingredient and stirred forming a wet granulation. The mixture is granulated using a suitable pharmaceutical mixer or granulator such as a planetary mixer or a high sear mixer then dried and calibrated.

The bioadhesive polymer, the sustained release polymer and compression excipients are then weighed and sieved. These ingredients were then added to the primary granulated mixture to form a final blending mixture, which was then further compressed using a suitable tablet press such as a rotative press.

The active principle that can be used in the method of the present invention are described above, as well as the particular diluents, binding agents, alkali metal alkylsulfates, bioadhesive polymers and sustained release polymers used in the present method.

In another embodiment the present invention provides a method of preparing a bioadhesive carrier, the method comprising;
a) mixing at least one active principle selected from:
   an antiviral, an analgesic, an anti-inflammatory, an antibiotic, an antiseptic, an antiemetic and mixtures thereof;
b) wetting said mixture with water;
c) drying and calibrating said mixture;
d) granulating said mixture to form primary granules;
e) blending said primary granules with at least one bioadhesive natural protein of vegetal origin and mixtures thereof and 5% to 80% by weight of at least one polymer that provides a sustained release of the active principle and at least one compression agent; and
f) compressing the blended mixture obtained In e).

In another embodiment the present invention provides a method of preparing a bioadhesive carrier, the method comprising:
a) mixing at least one active principle selected from:
   an antiviral, an analgesic, an anti-inflammatory, an antibiotic, an antiseptic, an antiemetic and mixtures thereof with 1 to 10 % by weight of an alkali metal alkylsulfate and 1 to 75 % by weight of a diluent;
b) wetting said mixture with 0.5 to 5 % by weight binding agent that has been solubilized;
c) drying and calibrating said mixture;
d) granulating said mixture to form primary granules;
e) blending said primary granules with at least one bioadhesive natural protein of vegetal origin and mixtures thereof and 5% to 80% by weight of at least one polymer that provides a sustained release of the active principle and at least one compression agent; and
f) compressing the blended mixture obtained in e),

In another aspect, the present invention also relates to a method of preparing a bioadhesive carrier, the method comprising:
a) mixing at least one active principle with a diluent, with at least one bioadhesive polymer and at least one sustained release polymer in a dry state to form a mixture;
b) compacting said mixture to form a sheet;
c) breaking the sheet to form a granulate;
d) blending said granulate with at least one compression agent; and
e) compressing the blended mixture obtained in d) into tablets.

In another aspect, the present invention also relates to a method of preparing a bioadhesive carrier, the method comprising:
a) mixing at least one active principle with a diluent, with at least one bioadhesive polymer, at least one sustained release polymer, with at least one compression agent in a dry state to form a mixture;
b) compressing the blended mixture obtained In a) into tablets.

In yet another aspect the present invention relates to the use of the mucosal bioadhesive slow release carrier according to the present invention for the manufacture of a medicament to treat mucosal diseases.

In this respect the mucosal diseases can be buccal diseases including herpes simplex virus 1 (HSV-1), herpes simplex virus (HSV-2), oral mucositis, oral Candidiasis, oral hairy leukoplakia, oral ulcers, salivary gland disturbance, altered oral flora (decreased bacterial flora), taste dysfunction, periodontitis, xerostomia, gastrointestinal mucositis causing secondary changes in oral status including inflammation, hygiene and dietary intact and oral pain. These diseases can be treated with at least one active principle or at least two different active principles.

In yet another aspect of the present invention, the mucosal diseases can be genital diseases including herpes simplex virus 2 (HSV-2), herpes simplex virus 1 (HSV-1) or human papilloma virus. These diseases can be treated with at least one active principle or at least two different active principles.

In yet another aspect the present invention relates to the use of the mucosal bioadhesive slow release carrier according to the invention for the manufacture of a medicament to treat herpes simplex virus 1 (HSV-1), herpes simplex virus 2 (HSV-2) Epstein-Barr virus, human papilloma virus, cytomegalovirus, variacella-Zostor virus, Kaposi's sarcoma duo to human herpes V8 and genital warts duo to human papilloma virus.

In another aspect the present invention provides a method of delivering at least one active principle to a mammal in need of said active principle comprising administering the bioadhesive slow release carrier of the present invention, which is described in detail above.

In yet another aspect, the present invention provides a method of treating a mucosal disease in a mammal in need of such treatment, said method comprising administering the bioadhesive slow release carrier ot the present invention, which is described in detail above. The mucosal diseases are described above.

A number of embodiments of the invention have been described. Nevertheless, it will be understood that various modifications may be made without departing from the spirit and scope of the invention.

### EXAMPLES

### Example 1: Preparation of the Bioadhesive Slow Release Carrier with different bioadhesive polymers

50 mg of acyclovir, 15% by weight microcrystalline cellulose and 4.5% by weight of sodium lauryl sulfate were weighed and sieved with a 0.7 to 2 mm sieve before premixing in a blender to provide the "initial mix."

At the same time, 0.4 % by weight polyvinylpyrrolidone was dissolved in purified water. The resulting solution was added to the initial mix and further stirred. The wetted mixture was then granulated using a pharmaceutical mixer or granulator such as a planetary mixer or high sear mixer and dried and then calibrated to 500 µm. The resulting pellets formed the "primary granules."

20% of mucoadhesive agent (pea protein or milk concentrate protein or carbopol 974 or chitosan), 15% hypromellose, 1% magnesium stearate and 0.4 % of colloidal silica were weighed and sieved using a 500 µm sieve. These ingredients were then added to the primary granulation to form the "final blending" mixture. The final blending mixture was then compressed using a tablet press such as a rotative press to produce the compressed carriers according to the invention.

The size of the tablets was about 8 mm in diameter. The dimension was chosen to be comfortable In the canine fossa.

The above-described method was suitable for the production of compressed carrier batches ranging from 2 to 23 kg.

### Example 2: In vitro evaluation of the adhesion-ability of the acyclovir bioadhesive slow release carrier

The adhesion ability of the bioadhesive slow release carrier according to the Example 1 was measured using a texturometer equipment. The acyclovir carrier was fixed on a plastic probe. The probe came down to the immerged stainless bench, stopped, and then came up.

Adhesion ability was expressed as the "adhesion force" (g), the maximum force needed to separate the tablet, fixed on the probe, from the stainless central parts.

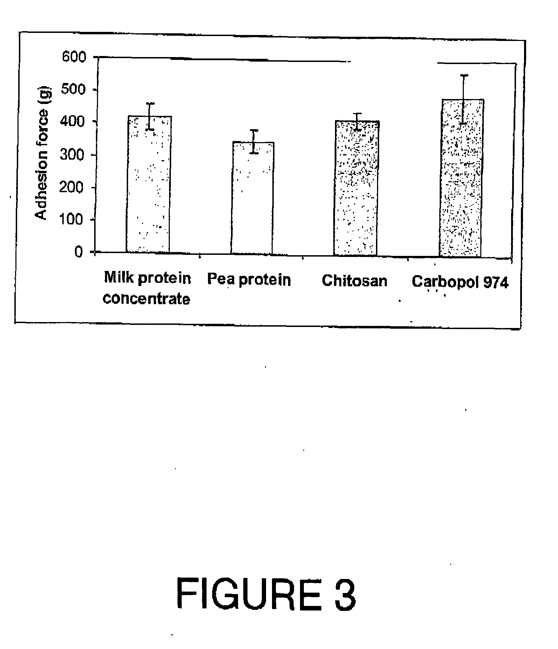

The results of the adhesion force for different mucoadhesive agent are presented in Figure 3.

The results have shown that the different mucoadhesive agents are able to confer similar adhesive properties and in particular that protein of vegetal origin like pea protein has adhesive properties.

## Claims

1. A mucosal bioadhesive slow release carrier comprising at least one active principle, at least one bioadhesive natural protein of vegetal origin and at least one polymer that provides sustained release of the active principle.

2. A mucosal bioadhesive slow release carrier comprising at least one active principle, a diluent, an alkali metal alkylsulfate, a binding agent, at least one bioadhesive natural protein of vegetal origin and at least one polymer that provides sustained release of the active principle.

3. The mucosal bioadhesive slow release carrier according to claims 1 or 2, wherein said at least one bioadhesive natural protein of vegetal origin is selected from the group of natural pea proteins, natural soy proteins, natural potato proteins, natural wheat proteins, glialdin proteins and mixtures thereof.

4. The mucosal bioadhesive slow release carrier according to claims 1 to 3 wherein said at least one bioadhesive natural protein of vegetal origin is a natural pea protein.

5. The mucosal bioadhesive slow release carrier according to Claim 2 wherein there is, 1 to 75 % by weight of a diluent and 1 to 10 % by weight of an alkali metal alkylsulfate, 0.5 to 5 % by weight of a binding agent and 5 to 80 % by weight of at least one bioadhesive natural proteins of vegetal origin and mixtures thereof and 5% to 80% by weight of at least one polymer that provides a sustained release of the active principle.

6. The mucosal bioadhesive slow release carrier according to any one of claims 1 to 4, wherein said at least one active principle is selected from: an antiviral, an analgesic, an anaesthetic, an antalgic, an anti-inflammatory, an antibiotic, an antiseptic, an antiemetic and mixtures thereof.

7. The mucosal bioadhesive slow release carrier according to any one of claims 1 to 6. wherein the alkali metal alkylsulfate is sodium lauryl sulfate.

8. The mucosal bioadhesive slow release carrier according to any one of claims 1 to 7, wherein the binding agent is polyvinylpyrrolidone.

9. The mucosal bioadhesive slow release carrier according to any one of claims 1 to 8, wherein the at least one natural protein of vegetal origin is in a concentration from 10 to 40% by weight.

10. The mucosal bioadhesive slow release carrier according to any one of claims 1 to 9, wherein the at least one polymer that provides a sustained release of the at least one active principle is hydrophilic and preferably a cellulose polymer.

11. The mucosal bioadhesive slow release carrier according to claim 10, wherein the cellulose polymer is hypromellose.

12. The mucosal bioadhesive slow release carrier according to any one of claims 1 to 11, wherein said at least one active principle is an antiviral selected from acyclovir, valacyclovir, zidovudine, ganciclovir, penciclovir, famciclovir, foscarnet, ribavirin, lamiduvine, amantadine, IFNa, cidofovir or rimantadine.

13. The mucosal bioadhesive slow release carrier according to claim 12, wherein said antiviral is acyclovir.

14. The mucosal bioadhesive slow release carrier according to claim 13, wherein said acyclovir is present in an amount from 10 to 500 mg in said carrier.

15. The mucosal bioadhesive slow release carrier according to claim 13, ' wherein said acyclovir is present in an amount from 50 to 100 mg in said carrier.

16. The mucosal bioadhesive slow release carrier according to any one of claims 1 to 11, wherein said at least one active principle is fentanyl or fentanyl citrate or sulfentanyl and is present in an amount of 50 to 1600 µg in said carrier.

17. The mucosal bioadhesive slow release carrier according to claim 16, wherein said fentanyl or fentanyl citrate or sulfentanyl and is present in an amount of from 200 to 1200 µg in said carrier.

18. The mucosal bioadhesive slow release carrier according to any one of claims 1 to 11, wherein said at least one active principle is an anti-inflammatory.

19. The mucosal bioadhesive slow release carrier according to claim 18, wherein said anti-inflammatory is a corticoid.

20. The mucosal bioadhesive slow release carrier according to any one of claims 1 to 11, wherein said at least one active principle is an antiviral that is active for herpes simplex viruses (HSV), varicella zoster virus (VZV), Epstein-Barr virus, human herpes virus 8, avian influenza, mumps, HIV, respiratory syncitial virus, influenza, parainfluenza and cytomegalovirus.

21. The mucosal bioadhesive slow release carrier according to any one of claims 1 to 20, wherein the at least one active principle is associated with at least two further active principles selected from: an antiviral, an antifungal, an analgesic, an anaesthetic, an antalgic, an antiemetic, a salivation agent, an antiseptic, an anti-inflammatory, an antibiotic and mixtures thereof.

22. The mucosal bioadhesive slow release carrier according to claim 6, wherein the antiseptic is sodium laurylsulfate in a minimum concentration of 2 to 10% by weight.

23. Use of the mucosal bioadhesive slow release carrier according to any one of claims 1 to 22 for the manufacture of a medicament to treat mucosal diseases.

24. Use according to claim 23, wherein the mucosal diseases are buccal diseases.

25. Use according to claim 24, wherein the buccal diseases are selected from: herpes simplex complex 1 (HSV-1), genital herpes (HSV-2), oral mucositis, oral Candidiasis, oral hairy leukoplakia, oral ulcers, salivary gland disturbance, altered oral flora (decreased bacterial flora), taste dysfunction, periodontitis, xerostomia, gastrointestinal mucositis causing secondary changes in oral status including inflammation, hygiene and dietary intact and oral pain.

26. Use of the mucosal bioadhesive slow release carrier according to claim 1 for the manufacture of a medicament to treat herpes simplex complex 1 (HSV-1), genital herpes (HSV-2) Epstein-Barr virus, human papilloma virus, cytomegalovirus, variacella-Zoster virus, Kaposi's sarcoma due to human herpes V 8 and genital warts due to human papilloma virus.

27. Use of the natural protein of vegetal origin as bioadhesive for the manufacture of a mucosel bioadhesive slow release carrier.

28. Use according to claim 27 wherein natural protein of vegetal origin are selected from the group of natural pea proteins, natural soy proteins, natural potato proteins, natural wheat proteins, giladin proteins ans mixtures thereof.
